## Europäisches Patentamt
## European Patent Office
## Office européen des brevets

(11) Numéro de publication: **0 139 545**
**B1**

(12) # FASCICULE DE BREVET EUROPÉEN

(45) Date de publication du fascicule du brevet:
13.01.88

(51) Int. Cl.⁴: **A 23 L 1/22** //
**A23L2/26, A23L2/38**

(21) Numéro de dépôt: **84401135.3**

(22) Date de dépôt: **05.06.84**

(54) Émulsions aqueuses d'anéthole et procédé pour leur préparation.

(30) Priorité: 06.06.83 FR 8309358
06.06.83 FR 8309360
15.09.83 FR 8314698

(43) Date de publication de la demande:
02.05.85 Bulletin 85/18

(45) Mention de la délivrance du brevet:
13.01.88 Bulletin 88/2

(84) Etats contractants désignés:
AT BE CH DE GB IT LI LU NL SE

(56) Documents cité:
FR-A-736 606
FR-A-972 738
FR-A-2 199 945
FR-A-2 280 326
FR-A-2 482 977
FR-A-2 483 455
GB-A-1 537 160
US-A-1 500 670
US-A-4 115 313

(73) Titulaire: **PERNOD RICARD, 142, Boulevard Haussmann, F-75008 Paris (FR)**

(72) Inventeur: **Clauzure, André, Evry- les- Châteaux, F-77166 Grisy- Suisnes (FR)**

(74) Mandataire: **Combe, André, CABINET BEAU DE LOMENIE 55, rue d'Amsterdam, F-75008 Paris (FR)**

EP 0 139 545 B1

## Description

La présente invention concerne de nouvelles émulsions stables d'anéthole ou d'huiles essentielles contenant de l'anéthole et un procéde pour leur préparation. Il s'agit d'"émulsion aqueuses", c'est-à-dire d'émulsions dont la phase continue est l'eau et la phase discontinue l'anéthole ou l'huile essentielle contenant de l'anéthole. Cependant, la phase continue de l'émulsion pourra avantageusement être constituée d'eau contenant une certaine proportion d'alcool, plus particulièrement de 5 à 25 % d'alcool. Ainsi, la présente invention a pour objet des émulsions stables d'anéthole ou d'huile essentielle contenant de l'anéthole, dans l'eau ou en milieu hydroalcoolique.

Lesdites émulsions contiennent, évidemment, de l'anéthole (ou des huiles essentielles contenant de l'anéthole), en quantité telle que cet anéthole n'est pas soluble dans le milieu dans lequel il est inclus.

Il est très intéressant de pouvoir disposer de telles émulsions, notamment en tant que boissons, stables dans le temps et avec la température.

On a donc besoin, d'une part, de disposer d'un agent émulsifiant possédant un ensemble de propriétés tel qu'il permette de réaliser une emulsion stable pendant plusieurs semaines et même plusieurs mois, qu'il soit acceptable dans le domaine alimentaire, qu'il soit compatible avec le produit à émulsionner et ne modifie pas le goût du produit émulsionné. De nombreux émulsifiants peuvent prétendre réunir ces diverses propriétés, cependant des recherches nouvelles ont permis de mettre en évidence que les saponines et les sels biliaires constituaient deux classes de produits utilisables comme émulsifiants selon l'invention.

L'utilisation des acides biliaires ou de leurs sels, en combinaison avec au moins un composé choisi parmi les mono- ou les di-glycérides, des phospholipides, des acides gras, des stéroïdes - un effet de synergie est recherché - pour la stabilisation de certains émulsions, a été décrite dans le brevet U.S. n° 4 115 313. Le document FR-A-2 483 455 décrit des émulsions stables d'huiles essentielles ou d'anéthole contenant un émulsifiant et un additif "antigel" naturel du groupe des terpènes, sesquiterpènes, aldéhydes et cétones.

D'autre part, l'anéthole étant un produit qui cristallise à une température de 20-22°C, il peut se faire que, lors du refroidissement des émulsions à des températures inférieures à environ +7°C, ledit anéthole cristallise dans ces émulsions. Ce phénomène de cristallisation est irréversible dans les émulsions hydroalcooliques ou difficilement réversible. Il est peu souhaitable pour l'aspect et les possibilités d'utilisation des émulsions. Il est donc également important de disposer d'un moyen d'éviter ou de retarder l'apparition de cette cristallisation de l'anéthole. Ce moyen est le second aspect de la présente invention. Pour assurer la stabilité, avec la température, des émulsions d'anéthole ou d'huiles essentielles contenant de l'anéthole, on fait intervenir, selon l'invention, au moins un triglycéride, dont le point de congélation est inférieur à 0°C.

L'invention concerne donc de nouvelles émulsions stables d'anéthole ou d'huiles essentielles contenant de l'anéthole, dans l'eau ou en milieu hydroalcoolique, caractérisées en ce qu'elles contiennent, en tant qu'agent émulsifiant, un composé choisi parmi les saponines et les sels biliaires, en une quantité d'au moins 2 % en poids par rapport à l'anéthole et, en tant qu'agent d'abaissement du point de cristallisation de l'anéthole, au moins un triglycéride, dont le point de congélation est inférieur à 0°C.

Les saponines (qui forment des émulsifiants utilisables dans l'invention) sont des substances glucosidiques d'origine végétale. Leur forme d'emploi privilégiée dans la présente invention est un extrait de plante, étant entendu que toute plante contenant des saponines peut être utilisée.

On citera à titre d'exemple des plantes référencées dans l'ouvrage du Conseil de l'Europe "Substances aromatisantes et sources naturelles de matières aromatisantes" 1981 (Maisonneuve).

| Plante | N° CEE | Partie utilisée | Classe | Principe à limite |
|---|---|---|---|---|
| Guajacum officinale | 220 | bois | N2 | |
| Polygala senega | 355 | racine | N2 | |
| Primula Eliator L | 364 | racine | N2 | coumarine |
| Herniaria glabra | 227 | herbe, partie aérienne | N3 | |
| Quillaja saponaria Molina | 391 | écorce | N3 | |
| Saponaria officinalis L. | 422 | racine | N3 | |
| Solanum dulcamara L. | 435 | tige | N3 | |
| Smilac utilis | 434 | racine | N4 | |

Les quantités de saponine à employer sont variables selon les produits à émulsifier et selon la nature de la saponine. On employerera au moins 2 % en poids de saponine par rapport au produit à émulsifier.

L'analyse de saponine contenue dans une émulsion selon l'invention est réalisée selon la méthode décrite dans: "CHEMISCHER-AUFBAU-PHYSIKALISCHE-EIGENSCHAFTEN UND UNTERSCHEIDUNGS-MERKMALE EINIGER POLYGALA-SAPONINE".
publiée dans
Deutsche Apotheker-Zeitung
108 Jahzgang N° 42 17.10.1968

2

0 139 545

Les sels biliaires utilisables selon l'invention sont essentiellement les sels des acides cholique, glycocholique, taurocholique ou désoxycholique. Ces sels sont utilisables à l'état de produits individualisés ou de préférence à l'état de mélanges de plusieurs sels.

On utilisera au moins 2 % en poids de sel biliaire par rapport au produit à émulsifier.

En mettant en oeuvre l'invention, on est parvenu à préparer des émulsions stables dans le temps d'anéthole ou d'huiles essentielles contenant de l'anéthole dans lesquelles la proportion d'anéthole peut varier d'environ 2 à environ 150 g/l, le milieu étant constitué d'eau ou d'un mélange d'eau et d'alcool contenant moins de 25 % en poids d'alcool et ce grâce à l'utilisation

- d'environ 0,04 à 3 g/l de saponines,
- ou d'environ 0,04 à 3 g/l de sels biliaires.

En ce qui concerne la stabilité avec la température, elle est obtenue grâce à l'intervention d'au moins un triglycéride.

Les triglycérides utilisables sont des produits connus; pour des raisons de législation sur les boissons il est généralement préférable d'employer non pas des triglycérides en tant que tels mais des produits naturels contenant des triglycérides. On emploiera donc des extraits naturels, contenant des triglycérides, qui présentent en outre les propriétés suivantes:

- être neutres ou peu aromatiques,
- être miscibles et compatibles avec l'anéthole, si nécessaire,
- présenter un bas point de congélation (inférieur à 0°C).

C'est ainsi que l'on peut utiliser les produits suivants: (isolément ou en mélange):

| huile de: | Température de congélation |
|---|---|
| - colza | - 4°C |
| - maïs | - 9°C |
| - pépin de raisin | -10°C |
| - tournesol | -16°C |
| - carthame | -15°C |

Les quantités de ces huiles à utiliser dépendront bien évidemment des quantités de triglycérides qui y sont contenues; on peut admettre l'emploi de 10 à 50 % en poids environ d'huile par rapport à l'anéthole présent dans la solution ou l'émulsion. Il est toutefois clair que ces quantités de triglycérides, si elles sont le plus souvent utilisées, ne sauraient constituer des limites strictes.

Le procédé de préparation des émulsions selon l'invention est de préférence le suivant:

- on mélange l'anéthole ou l'huile essentielle contenant de l'anéthole que l'on veut émulsifier avec une quantité convenable (telle que décrite ci-dessus) d'au moins un émulsifiant selon l'invention
- on verse ensuite l'eau ou le mélange hydroalcoolique de faible titre alcoolique sur le mélange précédemment obtenu et l'on soumet l'ensemble à l'action d'un homogénéisateur pour aboutir à la formation d'une émulsion stable.

Dans le procédé de préparation des émulsions selon l'invention, présenté ci-dessus, le triglycéride est très généralement et de préférence ajouté au mélange "initial" de l'anéthole ou de l'huile essentielle avec l'agent émulsifiant selon l'invention. L'eau ou le mélange hydroalcoolique n'est versé qu'ensuite sur ce mélange.

En utilisant les agents émulsifiants selon l'invention et de préférence le procédé décrit ci-dessus, il est possible de préparer:

- dans le domaine des boissons, des bases polyvalentes permettant l'élaboration, par dilution, de boissons sans alcool et/ou faiblement alcoolisées dont la puissance aromatique est indépendante du titre alcoolique.

Dans le cas des boissons alcoolisées, l'invention permet de préparer, dans l'état actuel de la législation française, une boisson émulsionnée sans recourir aux additifs, alors que l'usage des agents émulsifiants n'est pas autorisé.

Les exemples suivants illustrent l'invention; on a suivi le procédé décrit ci-dessus pour réaliser les émulsions stables (stabilité toujours supérieure à au moins 1 mois) suivantes:

**Exemple 1**

On prépare l'émulsion suivante:
pour 1 litre
- anéthole                                35   g
- huile nouvelle de colza                 12   g
- gomme résine Dammar                     3,5  g
- extrait aqueux de Polygala à 2,8 % de saponines:
48 ml eau q.s.p. 1 litre

Après avoir mélange ensemble l'anéthole, l'extrait aqueux de Polygala, l'huile nouvelle de colza et la gomme résine, on ajoute l'eau nécessaire pour obtenir 1 l d'émulsion concentrée. Après 30 s d'agitation violente à 20 000 tr/min, cette pré-émulsion est homogénéisée dans un homogénéisateur traditionnel.

Une boisson préparée à partir de cette émulsion contenant 2 g/l d'anéthole et titrant 20 % en volume d'alcool ne présente aucune cristallisation de l'anéthole après plusieurs mois à +2°C.

3

**Exemple 2**
| | | |
|---|---|---|
| - anéthole | 35 | g |
| - huile nouvelle de colza | 12 | g |
| - gomme résine Dammar | 3,5 | g |
| - extrait de Polygala alcoolisé à 60 % à 3,9 % de saponines | 38 | ml |
| eau-alcool q.s.p 1 l à 4 % d'alcool en volume | | |

**Exemple 3**
| | | |
|---|---|---|
| - anéthole | 35 | g |
| - huile de colza | 9 | g |
| - terpène d'orange | 3 | g |
| - gomme résine Dammar | 9 | g |
| - extrait Polygala alcoolisé à 60 % Polygala à 3,9 % de saponines | 38 | ml |
| eau-alcool q.s.p % final 16 % d'alcool | | |

**Exemple 4**
| | | |
|---|---|---|
| - anéthole | 35 | g |
| - huile de colza | 9 | g |
| - terpène | 3 | g |
| - gomme résine | 3,5 | g |
| - sels biliaires | 1 | g |
| - eau-alcool (à 4 % alcool) q.s.p | 1 | l |

**Exemple 5**
| | | |
|---|---|---|
| - anéthole | 35 | g |
| - nouvelle huile de colza | 12 | g |
| - extraits de polygala (à 2,8 % de saponine) | 48 | ml |
| - eau q.s.p | 1 | l |

A partir d'une émulsion concentrée, on peut obtenir une boisson titrant 2 g d'anéthole, sans alcool, et ne cristallisant pas à une température aussi basse que 2°C.

**Exemple 6**
| | | |
|---|---|---|
| - anéthole | 35 | g |
| - nouvelle huile de colza | 12 | g |
| - extrait alcoolisé de polygala à 3,9 % de saponine | 38 | ml |
| - mélange eau-alcool q.s.p | 1 | l (donnant naissance à une boisson contenant 4 % d'alcool). |

**Exemple 7**
| | | |
|---|---|---|
| - anéthole | 35 | g |
| - huile nouvelle de colza | 9 | g |
| - résine élémi | 3,5 | g |
| - sels biliaires | 1 | g |
| - eau q.s.p | 1 | l |

**Exemples 8, 9 et 10**

On a repris l'exemple 5 en remplaçant, poids pour poids, la nouvelle huile de colza par, d'une part, de l'huile de maïs et, d'autre part, de l'huile de noix et enfin de l'huile de pépin de raisin.
Dans chaque cas, à 2°C, on ne noterait aucune cristallisation de l'anéthole.

**Revendications** pour les Etats contractants: BE, CH, DE, GB, IT, LI, LU, NL, SE.

1. Emulsions stables d'anéthole ou d'huiles essentielles contenant de l'anéthole, dans l'eau ou en milieu hydroalcoolique, caractérisées en ce qu'elles contiennent, en tant qu'agent émulsifiant, un composé choisi parmi les saponines et les sels biliaires, en une quantité d'au moins 2 % en poids par rapport à l'anéthole, et,

en tant qu'agent d'abaissement du point de cristallisation de l'anéthole, au moins un triglycéride, dont le point de congélation est inférieur à 0°C.

2. Emulsions selon la revendication 1, caractérisées en ce que lesdites huiles essentielles contenant de l'anéthole sont des extraits végétaux concentrés.

3. Emulsions selon l'une des revendications 1 ou 2, caractérisées en ce que les saponines sont utilisées sous forme d'extraits végétaux concentrés, notamment de Polygala senega.

4. Emulsions selon l'une quelconque des revendications 1 à 3, caractérisées en ce qu'elles contiennent, en tant qu'agent émulsifiant, un mélange de plusieurs sels biliaires.

5. Emulsions selon l'une quelconque des revendications 1 à 4, caractérisées en ce qu'elles contiennent de 0,04 à 3 g/l de saponines ou de sels biliaires pour 2 à 150 g/l d'anéthole.

6. Emulsions selon l'une quelconque des revendications 1 à 5, caractérisées en ce qu'elles contiennent, en tant qu'agent d'abaissement du point de cristallisation de l'anéthole, au moins un produit naturel contenant des triglycérides.

7. Emulsions selon la revendication 6, caractérisées en ce que l'agent d'abaissement du point de cristallisation de l'anéthole est une huile choisie parmi les huiles de colza, maïs, pépin de raisin, tournesol, carthame.

8. Emulsions selon la revendication 7, caractérisées en ce qu'elles contiennent 10 à 50 % en poids d'huile par rapport à l'anéthole présent.

9. Emulsions selon l'une quelconque des revendications 1 à 8, caractérisées en ce qu'elles sont réalisées dans de l'eau ou dans une phase hydroalcoolique contenant de 5 à 25 % d'alcool éthylique.

10. Emulsions selon l'une quelconque des revendications précédentes, caractérisées en ce qu'elles contiennent en outre des additifs connus, tels que des gommes résines utilisées comme correcteur de densité.

11. Procédé pour la préparation d'une émulsion selon l'une quelconque des revendications précédentes, caractérisé en ce qu'il consiste à mélanger l'anéthole ou l'huile essentielle contenant de l'anéthole, l'émulsifiant choisi, le triglycéride ou produit naturel en contenant et l'eau ou le mélange hydroalcoolique, avec agitation convenable.

12. Procédé selon la revendication 11, caractérisé en ce qu'on mélange préalablement l'anéthole ou l'huile essentielle contenant de l'anéthole, l'émulsifiant choisi et le triglycéride ou produit naturel en contenant, puis que l'on verse sur ce mélange l'eau ou le mélange hydroalcoolique avec agitation convenable.


**Revendications** pour l'Etat contractant: AT.

1. Procédé pour la préparation d'émulsions stables d'anéthole ou d'huiles essentielles contenant de l'anéthole dans l'eau ou en milieu hydroalcoolique caractérisé en ce qu'il consiste à mélanger avec agitation convenable :
- l'anéthole ou l'huile essentielle contenant de l'anéthole;
- un agent émulsifiant choisi parmi les saponines et les sels biliaires intervenant en une quantité d'au moins 2 % en poids par rapport à l'anéthole ;
- un agent d'abaissement du point de cristallisation de l'anéthole: au moins un triglycéride dont le point de congélation est inférieur à 0°C;
- de l'eau ou un mélange hydroalcoolique.

2. Procédé selon la revendication 1, caractérisé en ce qu'il consiste à mélanger préalablement l'anéthole ou l'huile essentielle contenant de l'anéthole, l'émulsifiant choisi et le triglycéride puis à verser sur ce mélange l'eau ou le mélange hydroalcoolique.

3. Procédé selon l'une des revendications 1 ou 2, caractérisé en ce que lesdites huiles essentielles contenant de l'anéthole sont des extraits végétaux concentrés.

4. Procédé selon l'une quelconque des revendications 1 à 3, caractérisé en ce que les saponines sont utilisées sous forme d'extraits végétaux concentrés, notamment de Polygala senega.

5. Procédé selon l'une quelconque des revendications 1 à 4, caractérisé en ce que l'agent émulsifiant est un mélange de plusieurs sels biliaires.

6. Procédé selon l'une quelconque des revendications 1 à 5, caractérisé en ce que lesdites émulsions contiennent de 0,04 à 3 g/l de saponines ou de sels biliaires pour 2 à 150 g/l d'anéthole.

7. Procédé selon l'une quelconque des revendications 1 à 6, caractérisé en ce que lesdites émulsions contiennent en tant qu'agent d'abaissement du point de cristallisation de l'anéthole, au moins un produit naturel contenant des triglycérides.

8. Procédé selon la revendication 7, caractérisé en ce que l'agent d'abaissement du point de cristallisation de l'anéthole est une huile choisie parmi les huiles de colza, maïs, pépin de raisin, tournesol, carthame.

9. Procédé selon la revendication 8, caractérisé en ce que lesdites émulsions contiennent 10 à 50 % en poids d'huile par rapport à l'anéthole présent.

10. Procédé selon l'une quelconque des revendications précédentes calactérisé en ce que lesdites émulsions sont réalisées dans de l'eau ou dans une phase alcoolique contenant de 5 à 25 % d'alcool éthylique.

11. Procédé selon l'une quelconque des revendications précédentes, caractérisé en ce que lesdites émulsions contiennent en outre des additifs connus, tels que des gommes résines utilisées comme correcteur

de densité.

**Patentansprüche** für die Vertragsstaaten: BE, CH, DE, GB, IT, LI, LU, NL, SE.

1. Beständige Emulsionen von Anethol oder anetholhältigen etherischen Ölen in Wasser oder wässerig-alkoholischem Milieu, dadurch gekennzeichnet, daß sie als Emulgierungsmittel eine Verbindung, ausgewählt aus Saponinen und Gallensäuresalzen, in einer Menge von mindestens 2 Gew.-%, bezogen auf das Anethol, und als Mittel zum Herabsetzen des Kristallisationspunktes des Anethols mindestens ein Triglycerid, dessen Gefrierpunkt unter 0°C liegt, enthalten.

2. Emulsionen nach Anspruch 1, dadurch gekennzeichnet, daß die anetholhältigen etherischen Öle konzentrierte pflanzliche Extrakte sind.

3. Emulsionen nach einem der Ansprüche 1 oder 2, dadurch gekennzeichnet, daß die Saponine in Form von konzentrierten pflanzlichen Extrakten, insbesondere von Polygala senega, verwendet werden.

4. Emulsionen nach einem der Ansprüche 1 bis 3, dadurch gekennzeichnet, daß sie als Emulgierungsmittel eine Mischung von mehreren Gallensäuresalzen enthalten.

5. Emulsionen nach einem der Ansprüche 1 bis 4, dadurch gekennzeichnet, daß sie 0,04 bis 3 g/l an Saponinen oder Gallensäuresalzen pro 2 bis 150 g/l Anethol enthalten.

6. Emulsionen nach einem der Ansprüche 1 bis 5, dadurch gekennzeichnet, daß sie als Mittel zum Herabsetzen des Kristallisationspunktes des Anethols mindestens ein natürliches, Triglyceride enthaltendes Produkt enthalten.

7. Emulsionen nach Anspruch 6, dadurch gekennzeichnet, daß das Mittel zum Herabsetzen des Kristallisationspunktes des Anethols ein Öl, ausgewählt aus Raps-, Mais-, Weinbeerenkern-, Lackmus- und Safloröl, ist.

8. Emulsionen nach Anspruch 7, dadurch gekennzeichnet, daß sie 10 bis 50 Gew.-% Öl, bezogen auf das vorhandene Anethol, enthalten.

9. Emulsionen nach einem der Ansprüche 1 bis 8, dadurch gekennzeichnet, daß sie in Wasser oder in einer wässerig-alkoholischen Phase, enthaltend 5 bis 25 % Ethylalkohol, realisiert sind.

10. Emulsionen nach einem der vorhergehenden Ansprüche, dadurch gekennzeichnet, daß sie weiters bekannte Additiva, wie Gummiharze, als Dichtekorrigens enthalten.

11. Verfahren zur Herstellung einer Emulsion nach einem der vorhergehenden Ansprüche, dadurch gekennzeichnet, daß es darin besteht, daß das Anethol oder das anetholhältige etherische Öl, das gewählte Emulgierungsmittel, das Triglycerid oder das ein solches enthaltende natürliche Produkt und Wasser oder die wässerig-alkoholische Mischung unter geeignetem Rühren vermischt werden.

12. Verfahren nach Anspruch 11, dadurch gekennzeichnet, daß man zuerst das Anethol oder das anetholhältige etherische Öl, das gewählte Emulgierungsmittel und das Triglycerid oder das ein solches enthaltende natürliche Produkt vermischt und dann auf diese Mischung Wasser oder die wässerig-alkoholische Mischung unter geeignetem Rühren gießt.

**Patentansprüche** für den Vertragsstaat: AT.

1. Verfahren zur Herstellung von beständigen Emulsionen von Anethol oder anetholhältigen etherischen Ölen in Wasser oder wässerig-alkoholischem Milieu, dadurch gekennzeichnet, daß es im Mischen unter geeignetem Rühren von:
- dem Anethol oder anetholhältigen etherischen Öl,
- einem Emulgierungsmittel, ausgewählt aus Saponinen und Gallensäuresalzen, in einer Menge von mindestens 2 Gew.-%, bezogen auf das Anethol,
- einem Mittel zum Herabsetzen des Kristallisationspunktes des Anethols, u.zw. mindestens einem Triglycerid, dessen Gefrierpunkt unter 0°C liegt, und
- Wasser oder einer wässerig-alkoholischen Mischung besteht.

2. Verfahren nach Anspruch 1, dadurch gekennzeichnet, daß es darin besteht, daß man zuerst das Anethol oder das anetholhältige etherische Öl, das gewählte Emulgierungsmittel und das Triglycerid vermischt und dann auf diese Mischung Wasser oder die wässerig-alkoholische Mischung gießt.

3. Verfahren nach einem der Ansprüche 1 oder 2, dadurch gekennzeichnet, daß die anetholhältigen etherischen Öle konzentrierte pflanzliche Extrakte sind.

4. Verfahren nach einem der Ansprüche 1 bis 3, dadurch gekennzeichnet, daß die Saponine in Form von konzentrierten pflanzlichen Extrakten, insbesondere von Polygala senega, verwendet werden.

5. Verfahren nach einem der Ansprüche 1 bis 4, dadurch gekennzeichnet, daß das Emulgierungsmittel eine Mischung von mehreren Gallensäuresalzen ist.

6. Verfahren nach einem der Ansprüche 1 bis 5, dadurch gekennzeichnet, daß die Emulsionen 0,04 bis 3 g/l an Saponinen oder Gallensäuresalzen pro 2 bis 150 g/l Anethol enthalten.

7. Verfahren nach einem der Ansprüche 1 bis 6, dadurch gekennzeichnet, daß die Emulsionen als Mittel zum

6

Herabsetzen des Kristallisationspunktes des Anethols mindestens ein natürliches, Triglyceride enthaltendes Produkt enthalten.

8. Verfahren nach Anspruch 7, dadurch gekennzeichnet, daß das Mittel zum Herabsetzen des Kristallisationspunktes des Anethols ein Öl, ausgewählt aus Raps- Mais-, Weinbeerenkern-, Lackmus- und Safloröl, ist.

9. Verfahren nach Anspruch 8, dadurch gekennzeichnet, daß die Emulsionen 10 bis 50 Gew.-% Öl, bezogen auf das vorhandene Anethol, enthalten.

10. Verfahren nach einem der vorhergehenden Ansprüche, dadurch gekennzeichnet, daß die Emulsionen in Wasser oder in einer wässerig-alkoholischen Phase, enthaltend 5 bis 25 % Ethylalkohol, realisiert sind.

11. Verfahren nach einem der vorhergehenden Ansprüche, dadurch gekennzeichnet, daß die Emulsionen weiters bekannte Additiva, wie Gummiharze, als Dichtekorrigens enthalten.


**Claims** for the contracting states: BE, CH, DE, GB, IT, LI, LU, NL, SE.

1. Stable emulsions of anethole or of essential oils containing anethole, in water or in a water-alcohol medium, characterized in that they contain, as emulsifying agent, a compound selected from saponins and bile salts, in a quantity of at least 2 % by weight with respect to anethole, and as agent lowering the crystallization point of anethole, at least one triglyceride having a freezing point less than 0°C.

2. Emulsions according to claim 1, characterized in that said essential oils containing anethole are concentrated vegetable extracts.

3. Emulsions according to any one of claim 1 or 2, characterized in that the saponins are used in the form of concentrated vegetable extracts, particularly Polygala senega.

4. Emulsions according to any one of claims 1 to 3, characterized in that they contain, as emulsifying agent, a mixture of several bile salts.

5. Emulsions according to any one of claims 1 to 4, characterized in that they contain from 0.04 to 3 g/l of saponins or bile salts for 2 to 150 g/l of anethole.

6. Emulsions according to any one of claims 1 to 5, characterized in that they contain, as agent lowering the crystallization point of anethole, at least one natural product containing triglycerides.

7. Emulsions according to claim 6, characterized in that the agent lowering the crystallization point of anethole is an oil selected from oil of colza, corn, grape pip, sunflower, safflower.

8. Emulsions according to claim 7, characterized in that they contain 10 to 50 % by weight of an oil, with respect to the anethole.

9. Emulsion according to any one of claims 1 to 8, characterized in that they are made in water or in a water-alcohol phase containing 5 to 25 % of ethyl alcohol.

10. Emulsions according to any one of the preceding claims, characterized in that they further contain known additives, such as resin gums used as density corrector.

11. Process for the preparation of an emulsion according to any one of the preceding claims, characterized in that it consists in mixing the anethole or the essential oil containing anethole, with the chosen emulsifier, the triglyceride or a natural product containing the triglyceride, the water or the water-alcohol mixture, with appropriate stirring.

12. Process according to claim 11, characterized in that the anethole or the essential oil containing anethole is mixed beforehand with the chosen emulsifier and the triglyceride or a natural product containing triglyceride, then said water or the water-alcohol mixture is poured in said mixture with appropriate stirring.


**Claims** for the contracting state: AT.

1. Process for the preparation of stable emulsions of anethole or of essential oils containing anethole, in water or in a water-alcohol medium, characterized in that it consists in mixing with appropriate stirring:
- the anethole or the essential oil containing anethole;
- an emulsifying agent selected from saponins and bile salts in a quantity of at least 2 % by weight with respect to anethole;
- an agent lowering the crystallization point of anethole: at least one triglyceride having a freezing point less than 0°C;
- water or a water-alcohol mixture.

2. Process according to claim 1, characterized in that it consists in mixing beforehand the anethole or the essential oil containing anethole, with the chosen emulsifier and the triglyceride, then pouring in said mixture the water or the water-alcohol mixture.

3. Process according to any one of claims 1 or 2, characterized in that said essential oils containing anethole are concentrated vegetable extracts.

4. Process according to any one of claims 1 to 3, characterized in that the saponins are used in the form of concentrated vegetable extracts, in particular Polygala senega.

5. Process according to any one of claims 1 to 4, characterized in that the emulsifying agent is a mixture of several bile salts.

6. Process according to any one of claims 1 to 5, characterized in that said emulsions contain from 0.04 to 3 g/l of saponins or bile salts for 2 to 150 g/l of anethole.

7. Process according to any one of claims 1 to 6, characterized in that said emulsions contain as agent lowering the crystallization point of anethole, at least one natural product containing triglycerides.

8. Process according to claim 7, characterized in that the agent lowering the crystallization point of anethole is an oil selected from oil of colza, corn, grape pip, sunflower, safflower.

9. Process according to claim 8, characterized in that said emulsions contain 10 to 50 % by weight of an oil with respect to the present anethole.

10. Process according to any one of the preceding claims, characterized in that said emulsions are made in water or in a water-alcohol phase containing from 5 to 25 % of ethyl alcohol.

11. Process according to any one of the preceding claims, characterized in that said emulsions further contain known additives, such as resin gums used as density corrector.